(19) 

Europäisches
Patentamt
European
Patent Office
Office européen
des brevets

(11) **EP 3 594 887 A1**

(12) **EUROPEAN PATENT APPLICATION**

(43) Date of publication:
**15.01.2020 Bulletin 2020/03**

(51) Int Cl.:
***G06Q 30/02*** *(2012.01)*

(21) Application number: **19185284.7**

(22) Date of filing: **09.07.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **09.07.2018 GB 201811234**

(71) Applicant: **Vitargent (International) Biotechnology
Limited
Shatin, N.T. (HK)**

(72) Inventors:
- **CHEN, Xueping
  Shatin, N.T. (HK)**
- **CHEN, Zixiang
  Shatin, N.T. (HK)**
- **TAO, Wai Leung
  Shatin, N.T. (HK)**

(74) Representative: **J A Kemp LLP
14 South Square
Gray's Inn
London WC1R 5JJ (GB)**

(54) **COMPUTING SYSTEM AND METHOD FOR DETERMINING SAFETY INDEX OF PRODUCT**

(57) The present invention relates to computing system and method for determining a safety index of a product. The method includes: retrieving, by the computing system, a plurality of toxicity values of the product from a toxicity database, wherein the toxicity values correspond to a plurality types of the product respectively; retrieving, by the computing system, a plurality of weight values of the product from a weight database, wherein the weight values correspond to the types of the product respectively; and determining, by the computing system, the safety index of the product according to the toxicity values and the weight values.

EP 3 594 887 A1

## Description

### Field of the Invention

The present disclosure generally relates to computing system and method for determining a safety index of a product, and more particularly to computing system and method for determining a safety index of a consumer product.

### Background of the Invention

Consumer products, such as beverages and foods, health supplements, skin care and makeup cosmetics, can go through many processes in which potentially toxic chemicals can enter before reaching the consumer. The US Environmental Protection Agency tracks or regulates more than 100,000 chemicals (Substance Registry Services Fact Sheet, available at ofmpub.epa.gov/sor_internet/registry/substreg/educationalresources), and the toxicity of many of these chemicals have not been well studied, especially their total biological toxicity when combined with other chemicals. Ensuring the safety of product is a great challenge to the modern testing industry given the sheer number of potentially toxic chemicals and chemical combinations that can find their way into the end products.

To date, toxicity testing of consumables still largely relies on chemical-specific tests, especially chemical analysis. A review article relates to an overall view of the state-of-the-art and the technical knowhow concerning the extraction and chromatographic techniques for the determination of allowed ingredients in cosmetic products (Analytica Chimica Acta 915, (2016), pp. 1-26). While chemical-specific tests can be sensitive and precise, they can fail to detect unknown toxicants that are not intended to be specifically tested; this can allow unanticipated toxicants to go undetected. Even in cases where the chemical composition of a sample is known in detail, its effective toxicity cannot necessarily be reliably predicted due to the lack of knowledge concerning the effects of chemical mixtures. Practical experience with studies has shown that chemical-specific measurements identify true toxicity in unknown samples only about 20% of the time, which means up to 80% of toxicants are unidentified.

US publication No.: 2017/0356895 (which is incorporated herein by reference) discloses methods of determining whether a toxicant is present in a consumable product, which includes contacting a teleost embryo with an extract from a sample of the consumable product and determining whether the extract exerts a toxicity effect on the embryo. US publication No.: 2017/0356900 provides methods of determining whether a toxicant is present in a cosmetic product, which includes contacting a teleost embryo with a sample of the cosmetic product or an extract from a sample of the cosmetic product and determining whether the sample or the extract from the sample exerts a toxicity effect on the embryo.

However, these prior methods can only obtain information on toxic results of an individual testing sample but are not able to provide any indicative safety information for a product.

### Summary of the Invention

One embodiment of the present disclosure provides a method for determining a safety index of a product. The method includes: retrieving, by a computing system, a plurality of toxicity values of the product from a toxicity database, wherein the toxicity values correspond to a plurality types of the product respectively; retrieving, by the computing system, a plurality of weight values of the product from a weight database, wherein the weight values correspond to the types of the product respectively; determining, by the computing system, the safety index of the product according to the toxicity values and the weight values.

In some embodiments of the present disclosure, the method further includes: optionally performing a toxicity test to a plurality of samples of the types of the product to obtain the toxicity values; storing, by the computing system, the toxicity values in the toxicity database.

In some embodiments of the present disclosure, the toxicity test includes an acute toxicity test and the toxicity values are associated with lethal concentration 50% ($LC_{50}$) values.

In some embodiments of the present disclosure, the toxicity test includes a chronic toxicity test and the toxicity values are associated with estradiol equivalent concentration (EEQ) values.

In some embodiments of the present disclosure, the weight values include sales volume information.

In some embodiments of the present disclosure, the safety index of the product is determined based on following formula:

$$\mathrm{SI} = \sum_{h=1}^{x} \bar{T}_h \times \omega_h$$

wherein SI represents the safety index of the product, x represents number of the types of the product, $\overline{T}_h$ represents an average of the toxicity values of $h^{th}$ type of the product, and $\omega_h$ represents the weight value of $h^{th}$ type of the product.

In some embodiments of the present disclosure, $\overline{T}_h$ is determined based on following formula:

$$\bar{T}_h = \frac{1}{N_h} \sum_{i=1}^{N_h} T_{hi}$$

wherein $N_h$ represents number of samples of $h^{th}$ type of the product and $T_{hi}$ represents a toxicity test value of $i^{th}$ sample of $h^{th}$ type of the product.

In some embodiments of the present disclosure, $\overline{T}_h$ may be transformed to $\overline{T}_H$ based on following formulas so that the value may be set between [0, 100]:

$$T_{hi}^* = \frac{T_{hi} - \min\limits_{1 \le i \le N_h} T_{hi}}{\max\limits_{1 \le i \le N_h} T_{hi} - \min\limits_{1 \le i \le N_h} T_{hi}} \times 100\%$$

$$\bar{T}_H = \frac{1}{N_h} \sum_{i=1}^{N_h} T_{hi}^*$$

In some embodiments of the present disclosure, $\omega_h$ is determined based on following formula:

$$\omega_h = \frac{\sum_{i=1}^{N_h} C_{hi} P_{hi}}{\sum_{h=1}^{x} \sum_{i=1}^{N_h} C_{hi} P_{hi}}$$

wherein $N_h$ represents number of samples of $h^{th}$ type of the product, $C_{hi}$ represents an amount of $i^{th}$ sample of $h^{th}$ type of the product and $P_{hi}$ represents a rank weight of $i^{th}$ sample of $h^{th}$ type of the product.

In some embodiments of the present disclosure, the rank weights of the samples of $h^{th}$ type of the product correspond to sale volumes of the samples $h^{th}$ type of the product. More specifically, the rank weights may be decided based on the product popularity. The rank weights reflect the sales volume information to a certain extent.

Another embodiment of the present disclosure provides computing system for determining a safety index of a product. The computing system includes a processor and a memory. The memory stores instructions that, when being executed, cause the processor to: retrieve a plurality of toxicity values of the product from a toxicity database, wherein the toxicity values correspond to a plurality types of the product respectively; retrieve a plurality of weight values of the product from a weight database, wherein the weight values correspond to the types of the product respectively; determine the safety index of the product according to the toxicity values and the weight values.

In some embodiments of the present disclosure, a toxicity test may be optionally performed to a plurality of samples of the types of the product to obtain the toxicity values. The instructions, when being executed, further cause the processor to: store the toxicity values in the toxicity database.

In some embodiments of the present disclosure, the toxicity test includes an acute toxicity test and the toxicity values are associated with $LC_{50}$ values.

In some embodiments of the present disclosure, the toxicity test includes a chronic toxicity test and the toxicity values are associated with EEQ values.

In some embodiments of the present disclosure, the weight values include sales volume information.

In some embodiments of the present disclosure, the safety index of the product is determined based on following formula:

$$\text{SI} = \sum_{h=1}^{x} \bar{T}_h \times \omega_h$$

wherein SI represents the safety index of the product, x represents number of the types of the product, $\bar{T}_h$ represents an average of the toxicity values of $h^{th}$ type of the product, and $\omega_h$ represents the weight value of $h^{th}$ type of the product.

In some embodiments of the present disclosure, $\bar{T}_h$ is determined based on following formula:

$$\bar{T}_h = \frac{1}{N_h} \sum_{i=1}^{N_h} T_{hi}$$

wherein $N_h$ represents number of samples of $h^{th}$ type of the product and $T_{hi}$ represents a toxicity test value of $i^{th}$ sample of $h^{th}$ type of the product.

In some embodiments of the present disclosure, $\bar{T}_h$ may be transformed to $\bar{T}_H$ based on following formulas so that the value may be set between [0, 100]:

$$T_{hi}^* = \frac{T_{hi} - \min_{1 \le i \le N_h} T_{hi}}{\max_{1 \le i \le N_h} T_{hi} - \min_{1 \le i \le N_h} T_{hi}} \times 100\%$$

$$\bar{T}_H = \frac{1}{N_h} \sum_{i=1}^{N_h} T_{hi}^*$$

In some embodiments of the present disclosure, $\omega_h$ is determined based on following formula:

$$\omega_h = \frac{\sum_{i=1}^{N_h} C_{hi} P_{hi}}{\sum_{h=1}^{x} \sum_{i=1}^{N_h} C_{hi} P_{hi}}$$

wherein Nh represents number of samples of $h^{th}$ type of the product, Chi represents an amount of $i^{th}$ sample of $h^{th}$ type of the product and $P_{hi}$ represents a rank weight of $i^{th}$ sample of $h^{th}$ type of the product.

In some embodiments of the present disclosure, the rank weights of the samples of $h^{th}$ type of the product correspond to sale volumes of the samples $h^{th}$ type of the product. More specifically, the rank weights may be decided based on the product popularity. The rank weights reflect the sales volume information to a certain extent.

## Detailed Description of the Invention

In order to describe the manner in which advantages and features of the disclosure can be obtained, a description of the disclosure is rendered by reference to specific embodiments thereof, which are illustrated in the appended drawings. These drawings depict only exemplary embodiments of the disclosure and are not therefore to be considered limiting of its scope.

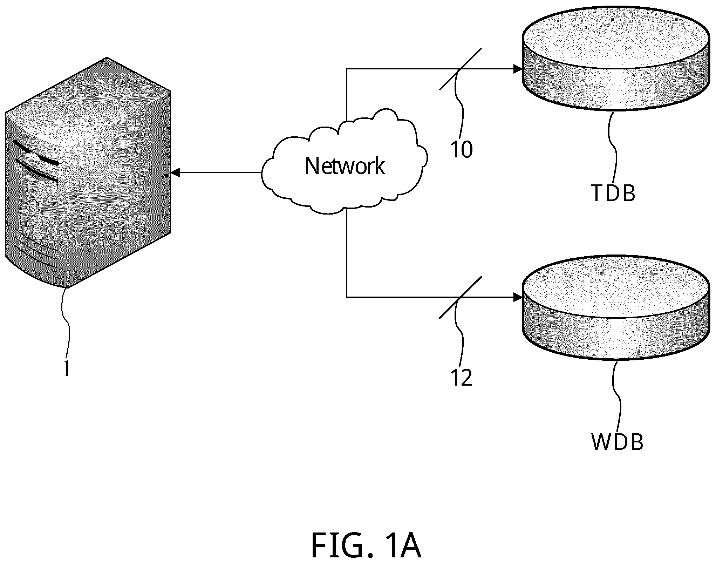

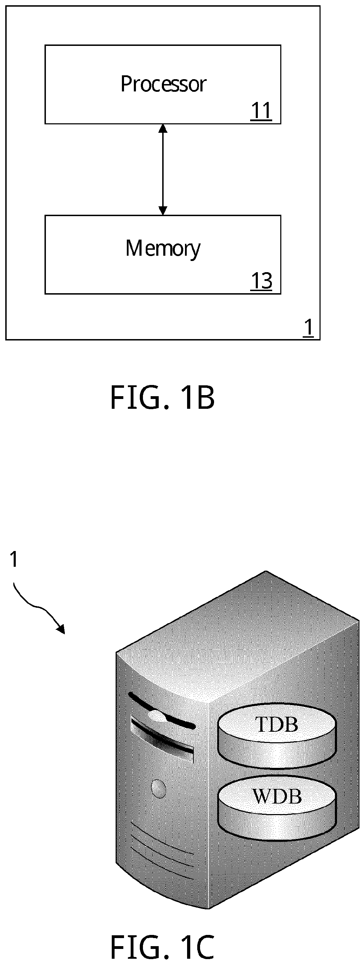

FIG. 1A illustrates a computing system 1 according to embodiments of the present disclosure. The computing system 1 connects to a toxicity database TDB and a weight database WDB via network. FIG. 1B is a block diagram of the computing system 1. The computing system 1 includes a processor 11 and a memory 13. The processor 11 and the memory 13 may be electrically coupled (e.g., electrically coupled through a communication bus). The memory 13 stores instructions that, when being executed, cause the processor to perform operations.

It should be noted that, in some embodiments, the toxicity database TDB and the weight database WDB may be included in the computing system 1. Please refer to FIG. 1C which illustrates the computing system 1 according to other embodiments of the present disclosure. In particular, the computing system 1 may include at least one non-volatile storage (not shown) which includes the toxicity database TDB and the weight database WDB. The processor 11 and the at least one non-volatile storage may be electrically coupled (e.g., electrically coupled through a communication bus). The interactions between the individual elements will be further described hereinafter.

In some embodiments, when a user needs to know if a product, especially edible product, is safe enough, the computing system 1 may be introduced to determine a safety index of the product. The safety index may be an indicator reflecting the safety situation (i.e., degree of safety) of the product.

In particular, the computing system 1 may retrieve a plurality of toxicity values 10 of the product from the toxicity database TDB. In some embodiments, when the computing system 1 connects with the toxicity database TDB via the network, the computing system 1 may further includes a transceiver (not shown) for retrieving the toxicity values 10 from the toxicity database TDB via the network. In some embodiments, when the computing system 1 includes the non-volatile storage which includes the toxicity database TDB, the computing system 1 may retrieve the toxicity values 10 from the toxicity database TDB via accessing the non-volatile storage.

More specifically, because the product may have several types and each type of the product may correspond to one toxicity value, thus, the product may correspond to more than one toxicity value. In other words, the toxicity values 10 of the product may correspond to the types of the product respectively.

Next, the computing system 1 may retrieve a plurality of weight values 12 of the product from the weight database TDB. In some embodiments, when the computing system 1 connects with the weight database WDB via the network, the computing system 1 may further includes a transceiver (not shown) for retrieving the weight values 12 from the weight database WDB via the network. In some embodiments, when the computing system 1 includes the non-volatile storage which includes the weight database WDB, the computing system 1 may retrieve the weight values 12 from the weight database WDB via accessing the non-volatile storage.

Similarly, because the product may have several types and each type of the product may correspond to one weight value, thus, the product may correspond to more than one weight value. In other words, the weight values 12 of the product may correspond to the types of the product respectively. Accordingly, after retrieving the toxicity values 10 and the weight values 12, the computing system 1 may determine the safety index of the product according to the toxicity values 10 and the weight values 12.

In some embodiments, the higher the toxicity value 10 is, the safer the corresponding type of the product is. Furthermore, the weight values 12 may include sales volume information. Each weight value 12 may be related to one sale volume information of the corresponding type of the product. The more popular the sale volume information indicates, the higher the corresponding weight value 12 is. Therefore, when one type of the product is with higher toxicity value 10 and higher weight value 12 (i.e., one type of the product is with less toxicity and is more popular in the market) may raise the safety index of the product.

In some embodiments, after retrieving the toxicity values 10 and the weight values 12, the safety index of the product may be determined based on following formula:

$$ \mathrm{SI} = \sum_{h=1}^{x} \bar{T}_h \times \omega_h $$

where SI represents the safety index of the product, x represents number of the types of the product, $\bar{T}_h$ represents the toxicity value 10 of $h^{th}$ type of the product, and $\omega_h$ represents the weight value 12 of $h^{th}$ type of the product.

In some embodiments, each type of the product may be tested with several samples. Accordingly, $\bar{T}_h$ is determined based on following formula:

$$ \bar{T}_h = \frac{1}{N_h} \sum_{i=1}^{N_h} T_{hi} $$

where Nh represents number of samples of $h^{th}$ type of the product and $T_{hi}$ represents a toxicity test value of $i^{th}$ sample of $h^{th}$ type of the product.

In some embodiments, different types of the product may correspond to different sales volume information. Accordingly, $\omega_h$, which may include sales volume information, is determined based on following formula:

$$\omega_h = \frac{\sum_{i=1}^{N_h} C_{hi} P_{hi}}{\sum_{h=1}^{x} \sum_{i=1}^{N_h} C_{hi} P_{hi}}$$

where $N_h$ represents number of samples of $h^{th}$ type of the product, Chi represents an amount of $i^{th}$ sample of $h^{th}$ type of the product and $P_{hi}$ represents a rank weight of $i^{th}$ sample of $h^{th}$ type of the product.

In some embodiments, before being used, the toxicity values 10 may be derived by performing a toxicity test to the types of the product. In particular, before using the toxicity values 10, the toxicity test may be performed to each sample of the types of the product to obtain the toxicity values 10, and then the toxicity values 10 of the types of the product may be stored in the toxicity database TDB for later use. The toxicity test may include an acute toxicity test and chronic toxicity test which may be achieved by contacting a teleost embryos with a testing type of product to obtain lethal concentration 50% ($LC_{50}$) and estradiol equivalent concentration (EEQ) values, respectively.

More specifically, the teleost embryos that can be used in the present disclosure can be of various freshwater, brackish water, or saltwater (marine water) species of fish, including, without limitation, fish of the *Oryzias* genus, the *Danio* genus and the *Pimephales* genus. Fish in the *Oryzias* genus belong to the *Adrianichthyidae* family and include, for example, *Oryzias melastigma* (alternative name *Oryzias dancena*) (marine or brackish medaka), *Oryzias latipes* (Japanese medaka), *Oryzias celebensis, Oryzias marmoratus, Oryzias matanensis, Oryzias nigrimas* (black buntingi), *Oryzias orthognathus* (buntingi), and *Oryzias profundicola.* Fish in the *Danio* genus belong to the *Cyprinidae* family and include, for example, *Danio rerio* (zebrafish), *Danio albolineatus, Danio abolineatus, Danio choprae, Danio dangila, Danio erythromicron, Danio feegradei, Danio kerri, Danio kyathit, Danio margaritatus, Danio meghalayensis, Danio nigrofasciatus,* and *Danio roseus.* Fish in the *Pimephales* genus belong to the Cyprinidae family and include *Pimephales notatus* (bluntnose minnow), *Pimephales promelas* (fathead minnow), *Pimephales tenellus* (slim minnow), and *Pimephales vigilax* (bullhead minnow).

In particular embodiments, the fish embryos are Japanese or brackish medaka fish, zebrafish or fathead minnow embryos. In some embodiments, the fish embryos can be transgenic or non-transgenic. It should be noted that the embodiments of the teleost embryos are disclosed in US publication No.: 2017/0356895 and US publication No.: 2017/0356900 (which are incorporated herein by reference).

In some embodiments, the product of the present disclosure can be measured for acute toxic effects or chronic toxic effects such as mortality and target gene expression level on a whole organism level. Various endpoints such as coagulation of eggs and embryos, failure to develop somites, lack of heart-beat as well as non-detachment of the tail from the yolk are indicative of acute toxicity. These endpoints can be recorded and used for the calculation of an $LC_{50}$ value of a product. Induced expression of target genes such *choriogenin* H expression in *choriogenin* H- green fluorescence protein genetically modified fish embryos are indicative of chronic toxicity. The fluorescence intensity can be measured as endpoint for the calculation of an EEQ value of a product. The embodiments of the toxicity testing are those disclosed in US publication No.: 2017/0356895 and US publication No.: 2017/0356900.

In addition, the toxicity values 10 of the present disclosure may be associated with $LC_{50}$ values and EEQ values of the product. In some embodiments, the toxicity test values for calculating the toxicity values 10 may be the $LC_{50}$ values. In particular, because a sample with higher $LC_{50}$ value indicates that the sample is safer, the $LC_{50}$ values may be used as the toxicity test values directly. Accordingly, the higher the $LC_{50}$ values (i.e., the toxicity test values) are, the higher the calculated toxicity values 10 are. Thus, it indicates that the corresponding types of the product are safer.

In some embodiments, the toxicity test values for calculating the toxicity values 10 may be a plurality of EEQ-inverse values transformed from the EEQ values. In particular, because a sample with lower EEQ value indicates that the sample is safer, the EEQ values may be transformed to the EEQ-inverse values before being used as the toxicity test values. More specifically, in these embodiments, the EEQ-inverse values may be used as the toxicity test values. Accordingly, the lower the EEQ values are, the higher the EEQ-inverse values (i.e., the toxicity test values) are. Subsequently, the higher the toxicity test values are, the higher the calculated toxicity values 10 are. Thus, it indicates that the corresponding types of the product are safer.

For ease of understanding the present disclosure, edible oil will be demonstrated as an example of the product hereinafter. In some embodiments, the edible oil may be classified under five types, namely: 1st: peanut oil C1; 2nd: corn oil C2; 3rd: olive oil C3; 4th: canola oil C4; and 5th: other oil C5. The 1st type of the edible oil (i.e., peanut oil C1) has twelve oil test samples. The 2nd type of the edible oil (i.e., corn oil C2) has eight oil test samples. The 3rd type of the edible oil (i.e., olive oil C3) has fifty-three oil test samples. The 4th type of the edible oil (i.e., canola oil C4) has twenty-four oil test samples. The 5th type of the edible oil (i.e., other oil C5) has eighteen oil test samples.

Further, the mentioned toxicity test may be performed to each oil test sample of the types of the edible oil for obtaining toxicity values. Then, the toxicity values may be stored in the computing system 1. In detail, the toxicity value of the peanut oil C1, which has twelve oil test samples, may be determined based on following formula:

$$\bar{T}_{peanut\ oil} = \frac{1}{12}\sum_{i=1}^{12} T_{sample\ i}$$

where $\bar{T}_{peanut\ oil}$ represents the toxicity value of the peanut oil C1 and the $T_{sample\ i}$ represents a toxicity test value of $i^{th}$ oil test sample of the peanut oil C1.

The toxicity value of the corn oil C2, which has eight oil test samples, may be determined based on following formula:

$$\bar{T}_{corn\ oil} = \frac{1}{8}\sum_{i=1}^{8} T_{sample\ i}$$

where $\bar{T}_{corn\ oil}$ represents the toxicity value of the corn oil C2 and the $T_{sample\ i}$ represents a toxicity test value of $i^{th}$ oil test sample of the corn oil C2.

The toxicity value of the olive oil C3, which has fifty-three oil test samples, may be determined based on following formula:

$$\bar{T}_{olive\ oil} = \frac{1}{53}\sum_{i=1}^{53} T_{sample\ i}$$

where $\bar{T}_{olive\ oil}$ represents the toxicity value of the olive oil C3 and the $T_{sample\ i}$ represents a toxicity test value of $i^{th}$ oil test sample of the olive oil C3.

The toxicity value of the canola oil C4, which has twenty-four oil test samples, may be determined based on following formula:

$$\bar{T}_{canola\ oil} = \frac{1}{24}\sum_{i=1}^{24} T_{sample\ i}$$

where $T_{canola\ oil}$ represents the toxicity value of the canola oil C4 and the $T_{sample\ i}$ represents a toxicity test value of $i^{th}$ oil test sample of the canola oil C4.

The toxicity value of the other oil C5, which has eighteen oil test samples, may be determined based on following formula:

$$\bar{T}_{other\ oil} = \frac{1}{18}\sum_{i=1}^{18} T_{sample\ i}$$

where $\bar{T}_{other\ oil}$ represents the toxicity value of the other oil CC and the $T_{sample\ i}$ represents a toxicity test value of $i^{th}$ oil test sample of the other oil C5.

It should be noted that the safety of the edible oil may be only related with the $LC_{50}$ values. Accordingly, the $LC_{50}$ values may be directly used as the toxicity test values for the toxicity values of the types of the edible oil.

In some embodiments, the toxicity values $\bar{T}_{peanut\ oil}$, $\bar{T}_{corn\ oil}$, $\bar{T}_{olive\ oil}$, $\bar{T}_{canola\ oil}$ and $\bar{T}_{other\ oil}$ of peanut oil C1, corn oil C2, olive oil C3, canola oil C4 and other oil C5 may be calculated as the following table:

| Types | C1 peanut oil | C2 corn oil | C3 olive oil | C4 canola oil | C5 other oil |
|---|---|---|---|---|---|
| Toxicity Value | 154.85 | 325.72 | 131.85 | 243.26 | 211.83 |

7

Further, the weight values $\omega_{peanut\ oil}$, $\omega_{corn\ oil}$, $\omega_{olive\ oil}$, $\omega_{canola\ oil}$ and $\omega_{other\ oil}$ of the peanut oil C1, corn oil C2, olive oil C3, canola oil C4 and other oil C5 may be determined for calculating the safety index. In detail, before determining the weight values, a variable Q for each type of the edible oil may be determined first. More specifically, in some embodiments, as for peanut oil C1 which may have fourteen peanut oil product samples (which may be decided based on number of different peanut oil products available in chosen supermarkets), there may be a variable $Q_{peanut}$ determined based on the following formula:

$$Q_{peanut} = \sum_{i=1}^{14} C_i P_i$$

where $C_i$ represents an amount of $i^{th}$ oil product sample of the peanut oil C1 and $P_i$ represents a rank weight of $i^{th}$ oil product sample of the peanut oil C1. In some embodiments, the amount of $i^{th}$ oil product sample of the peanut oil C1 may be the capacity (e.g., 100ml) of $i^{th}$ oil product sample of the peanut oil C1. The rank weight of $i^{th}$ oil product sample of the peanut oil C1 may be related to sale volume (i.e., market popularity) of $i^{th}$ oil product sample of the peanut oil C1. In other words, the more popular $i^{th}$ oil product sample is, the greater the corresponding rank weight is.

As for corn oil C2 which has seven corn oil product samples, there may be a variable $Q_{corn}$ determined based on the following formula:

$$Q_{corn} = \sum_{i=1}^{7} C_i P_i$$

where $C_i$ represents an amount of $i^{th}$ oil product sample of the corn oil C2 and $P_i$ represents a rank weight of $i^{th}$ oil product sample of the corn oil C2. In some embodiments, the amount of $i^{th}$ oil product sample of the corn oil C2 may be the capacity of $i^{th}$ oil product sample of the corn oil C2. The rank weight of $i^{th}$ oil product sample of the corn oil C2 may be related to the sale volume (i.e., market popularity) of $i^{th}$ oil product sample of the corn oil C2. In other words, the more popular $i^{th}$ oil product is sample, the greater the corresponding rank weight is.

As for olive oil C3 which has forty-nine olive oil product samples, there may be a variable $Q_{olive}$ determined based on the following formula:

$$Q_{olive} = \sum_{i=1}^{49} C_i P_i$$

where $C_i$ represents an amount of $i^{th}$ oil product sample of the olive oil C3 and $P_i$ represents a rank weight of $i^{th}$ oil product sample of the olive oil C3. In some embodiments, the amount of $i^{th}$ oil product sample of the olive oil C3 may be the capacity of $i^{th}$ oil product sample of the olive oil C3. The rank weight of $i^{th}$ oil product sample of the olive oil C3 may be related to the sale volume (i.e., market popularity) of $i^{th}$ oil product sample of the olive oil C3. In other words, the more popular $i^{th}$ oil product sample is, the greater the corresponding rank weight is.

As for canola oil C4 which has nineteen canola oil product samples, there may be a variable $Q_{canola}$ determined based on the following formula:

$$Q_{canola} = \sum_{i=1}^{19} C_i P_i$$

where $C_i$ represents an amount of $i^{th}$ oil product sample of the canola oil C4 and $P_i$ represents a rank weight of $i^{th}$ oil product sample of the canola oil C4. In some embodiments, the amount of $i^{th}$ oil product sample of the canola oil C4 may be the capacity of $i^{th}$ oil product sample of the canola oil C4. The rank weight of $i^{th}$ oil product sample of the canola oil C4 may be related to the sale volume (i.e., market popularity) of $i^{th}$ oil product sample of the canola oil C4. In other words, the more popular $i^{th}$ oil product sample is, the greater the corresponding rank weight is.

As for other oil C5 which has twenty-nine other oil product samples, there may be a variable $Q_{other}$ determined based on the following formula:

$$Q_{other} = \sum_{i=1}^{29} C_i P_i$$

where $C_i$ represents an amount of $i^{th}$ oil product sample of the other oil C5 and $P_i$ represents a rank weight of $i^{th}$ oil product sample of the other oil C5. In some embodiments, the amount of $i^{th}$ oil product sample of the other oil C5 may be the capacity of $i^{th}$ oil product sample of the other oil C5. The rank weight of $i^{th}$ oil product sample of the other oil C5 may be related to the sale volume (i.e., market popularity) of $i^{th}$ oil product sample of the other oil C5. In other words, the more popular $i^{th}$ oil product sample is, the greater the corresponding rank weight is.

Next, when $Q_{total}$ represents sum of $Q_{peanut}$, $Q_{corn}$, $Q_{olive}$, $Q_{canola}$ and $Q_{other}$, the weight value $\omega_{peanut\ oil}$ may be determined based on the following formula:

$$\omega_{peanut\ oil} = \frac{Q_{peanut\ oil}}{Q_{total}}$$

The weight value $\omega_{corn\ oil}$ may be determined based on the following formula:

$$\omega_{corn\ oil} = \frac{Q_{corn\ oil}}{Q_{total}}$$

The weight value $\omega_{olive\ oil}$ may be determined based on the following formula:

$$\omega_{olive\ oil} = \frac{Q_{olive\ oil}}{Q_{total}}$$

The weight value $\omega_{canola\ oil}$ may be determined based on the following formula:

$$\omega_{canola\ oil} = \frac{Q_{canola\ oil}}{Q_{total}}$$

The weight value $\omega_{other\ oil}$ may be determined based on the following formula:

$$\omega_{other\ oil} = \frac{Q_{other\ oil}}{Q_{total}}$$

It should be noted that, the mentioned weight values may be calculated based on a general formula as follow:

$$\omega_h = \frac{\sum_{i=1}^{N_h} C_{hi} P_{hi}}{\sum_{h=1}^{x} \sum_{i=1}^{N_h} C_{hi} P_{hi}}$$

wherein $N_h$ represents number of oil product sample of $h^{th}$ type oil of the edible oil, $C_{hi}$ represents an amount of $i^{th}$ oil product sample of $h^{th}$ type oil of the edible oil and $P_{hi}$ represents a rank weight of $i^{th}$ oil product sample of $h^{th}$ type oil of the edible oil.

In some embodiments, the weight values $\omega_{peanut\ oil}$, $\omega_{corn\ oil}$, $\omega_{olive\ oil}$, $\omega_{canola\ oil}$ and $\omega_{other\ oil}$ of peanut oil C1, corn oil C2, olive oil C3, canola oil C4 and other oil C5 may be calculated as the following table:

| Types | C1 peanut oil | C2 corn oil | C3 olive oil | C4 canola oil | C5 other oil |
|---|---|---|---|---|---|
| Weight Value | 0.28 | 0.19 | 0.24 | 0.22 | 0.07 |

According to the toxicity values and the weight values of the peanut oil C1, corn oil C2, olive oil C3, canola oil C4 and other oil C5, a safety index SI may be determined based on the following formula:

$$SI = \sum \bar{T}_h \times \omega_h$$

where h may be selected from the types of the edible oil. In other words, h may be selected from peanut oil, corn oil, olive oil, canola oil and other oil. Based on the formula and the values in the tables, the safety index SI of edible oil may be calculated as:

$$0.28 \times 154.85 + 0.19 \times 325.72 + 0.24 \times 131.85 + 0.22 \times 243.26 + 0.07 \times 211.83 = 205.23$$

In some embodiments, the rank weights correspond to sale volumes of the products. More specifically, the rank weights may be decided based on the product popularities which reflect the sales volume information to a certain extent. In particular, the rank weights may be in linear descending order and be assigned one by one to the products which are ranked by the product popularities.

For example, because the peanut oil C1 has fourteen peanut oil product samples, there may be fourteen rank weights in linear descending order or in linear function inverse order. The fourteen peanut oil product samples may be ranked by the sale volumes. Accordingly, the fourteen rank weights, which are in linear descending order or linear function inverse order, may be assigned one by one to the peanut oil product samples which are ranked by the sale volumes. Basically, the higher rank weight may be assigned to the peanut oil product sample with the higher sale volume.

In some embodiments, the rank weights may be in linear descending order and be assigned one by one to groups of the product samples which are ranked by the product popularities. For example, because the peanut oil C1 has fourteen peanut oil product samples and, for example, two product samples may be grouped into one group, there may be seven rank weights, which correspond to seven groups of the peanut oil product samples, in linear descending order. In particular, the fourteen peanut oil product samples may be ranked by the product popularities, and then grouped into seven groups. Accordingly, the seven rank weights in linear descending order may be assigned one by one to the groups of the peanut oil product samples. Basically, the higher rank weight may be assigned to the group of the peanut oil product samples with the higher sale volume and the peanut oil product samples in the same group have the same rank weight.

In some embodiments, for the user to understand the safety index more easily with a score from 0 to 100, toxicity value $\bar{T}_h$ may be transformed to $\bar{T}_H$ based on following formulas:

$$T_{hi}^* = \frac{T_{hi} - \min\limits_{1 \le i \le N_h} T_{hi}}{\max\limits_{1 \le i \le N_h} T_{hi} - \min\limits_{1 \le i \le N_h} T_{hi}} \times 100\%$$

Furthermore, $\bar{T}_H$ may be set as:

$$\bar{T}_H = \frac{1}{N_h} \sum_{i=1}^{N_h} T_{hi}^*$$

For example, based on the toxicity values of peanut oil C1, corn oil C2, olive oil C3, canola oil C4 and other oil C5 listed in the table, the toxicity values may be standardized between 0 to 100 by a threshold value 172 as the following table:

| Types | C1 peanut oil | C2 corn oil | C3 olive oil | C4 canola oil | C5 other oil |
|---|---|---|---|---|---|
| Standardized Toxicity Value | 73.22 | 100 | 41.05 | 76.56 | 62.60 |

Accordingly, based on the formula of calculating the safety index and the values in the tables, the safety index SI of edible oil may be calculated as:

$$0.28 \times 73.22 + 0.19 \times 100 + 0.24 \times 41.05 + 0.22 \times 76.56 + 0.07 \times 62.60 = 70.58$$

For example, the toxicity values may be standardized between 0 to 100 by a threshold value 270 as the following table:

| Types | C1 peanut oil | C2 corn oil | C3 olive oil | C4 canola oil | C5 other oil |
|---|---|---|---|---|---|
| Standardized Toxicity Value | 52.7 | 93.21 | 33.98 | 67.05 | 55.58 |

Accordingly, based on the formula of calculating the safety index and the values in the tables, the safety index SI of edible oil may be calculated as:

$$0.28 \times 52.7 + 0.19 \times 93.21 + 0.24 \times 33.98 + 0.22 \times 67.05 + 0.07 \times 55.58 = 59.26$$

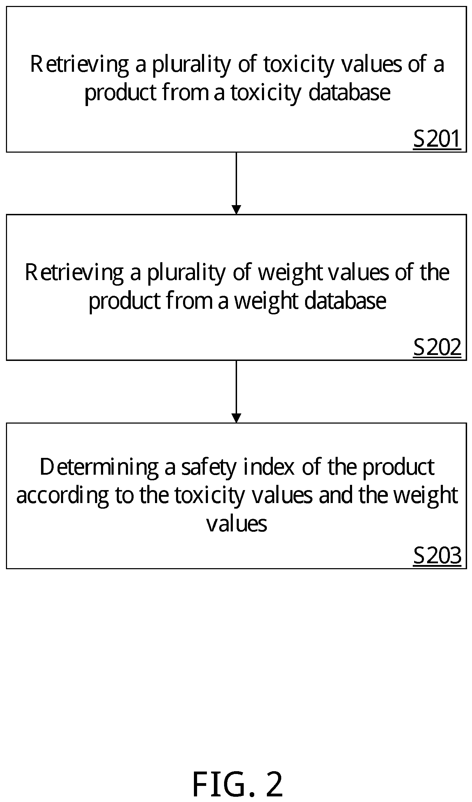

FIG. 2 is a flowchart showing a method for determining a safety index of a product in accordance with some embodiments of the present disclosure. The method of some embodiments may be executed by a computing system (e.g., the computing system of the aforesaid embodiments). Detailed operations of the method are as follows.

Operation S201 is executed, by the computing system, to retrieve a plurality of toxicity values of the product from a toxicity database. The toxicity values correspond to a plurality types of the product respectively. Operation S202 is executed, by the computing system, to retrieve a plurality of weight values of the product from a weight database. The weight values correspond to the types of the product respectively. Operation S203 is executed, by the computing system, to determine the safety index of the product according to the toxicity values and the weight values.

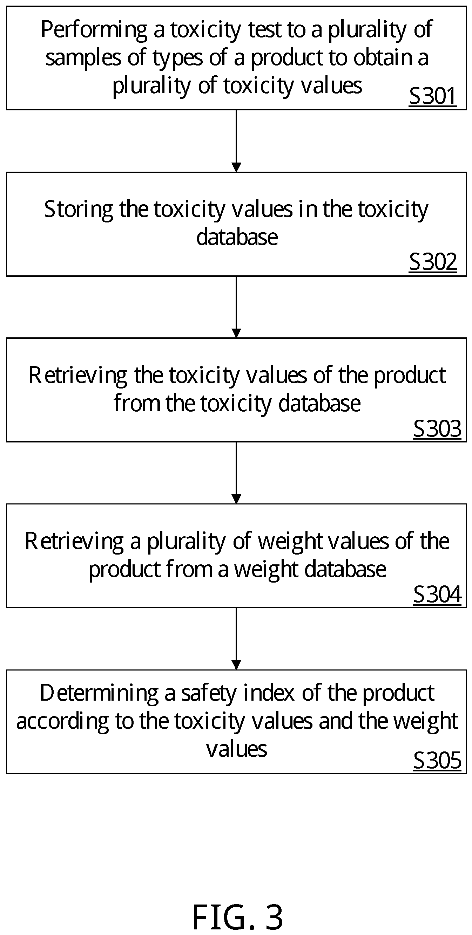

FIG. 3 is a flowchart showing a method for determining a safety index of a product in accordance with some embodiments of the present disclosure. The method of some embodiments may be executed by a computing system (e.g., the computing system of the aforesaid embodiments). Detailed operations of the method are as follows.

In some embodiments, operation S301 may be optional. In particular, a toxicity test may be optionally performed to a plurality of samples of types of a product to obtain a plurality of toxicity values. The toxicity values correspond to the types of the product. In some embodiments, the toxicity test includes an acute toxicity test and the toxicity values are associated with $LC_{50}$ values. In some embodiments, the toxicity test includes a chronic toxicity test and the toxicity values are associated with EEQ values. Then, operation S302 is executed, by the computing system, to store the toxicity values in a toxicity database.

Operation S303 is executed, by the computing system, to retrieve the toxicity values of the product from the toxicity database. Operation S304 is executed, by the computing system, to retrieve a plurality of weight values of the product from a weight database. The weight values correspond to the types of the product respectively. In some embodiments, the weight values include sales volume information. Operation S305 is executed, by the computing system, to determine the safety index of the product according to the toxicity values and the weight values.

In some embodiments, the safety index of the product is determined based on following formula:

$$SI = \sum_{h=1}^{x} \overline{T}_h \times \omega_h$$

where SI represents the safety index of the product, x represents number of the types of the product, $\overline{T}_h$ represents the toxicity value of $h^{th}$ type of the product, and $\omega_h$ represents the weight value of $h^{th}$ type of the product.

In some embodiments, $\overline{T}_h$ is determined based on following formula:

$$\overline{T}_h = \frac{1}{N_h} \sum_{i=1}^{N_h} T_{hi}$$

where $N_h$ represents number of samples of $h^{th}$ type of the product and $T_{hi}$ represents a toxicity test value of $i^{th}$ sample of $h^{th}$ type of the product.

In some embodiments, $\overline{T}_h$ may be transformed to $\overline{T}_H$ based on following formulas so that the value may be set between [0, 100]:

$$T_{hi}^* = \frac{T_{hi} - \min_{1 \le i \le N_h} T_{hi}}{\max_{1 \le i \le N_h} T_{hi} - \min_{1 \le i \le N_h} T_{hi}} \times 100\%$$

$$\overline{T}_H = \frac{1}{N_h} \sum_{i=1}^{N_h} T_{hi}^*$$

In some embodiments, $\omega_h$ is determined based on following formula:

$$\omega_h = \frac{\sum_{i=1}^{N_h} C_{hi} P_{hi}}{\sum_{h=1}^{x} \sum_{i=1}^{N_h} C_{hi} P_{hi}}$$

where $N_h$ represents number of samples of $h^{th}$ type of the product, $C_{hi}$ represents an amount of $i^{th}$ sample of $h^{th}$ type of the product and $P_{hi}$ represents a rank weight of $i^{th}$ sample of $h^{th}$ type of the product.

Although the present disclosure and its advantages have been described in detail, it should be understood that various changes, substitutions and alterations can be made herein without departing from the spirit and scope of the disclosure as defined by the appended claims. For example, the processors mentioned in the above embodiments may be a central processing unit (CPU), other hardware circuit elements capable of executing relevant instructions, or combination of computing circuits that shall be well-appreciated by those skilled in the art based on the above disclosures. Moreover, the memory mentioned in the above embodiments may be memories, such as ROM, RAM, etc., for storing data. Further, the transceiver mentioned in the above embodiments may be a combination of a network data transmitter and a network data receiver. However, it is not intended to limit the hardware implementation embodiments of the present disclosure.

Furthermore, the scope of the present application is not intended to be limited to the particular embodiments of the process, machine, manufacture, composition of matter, means, methods and steps described in the specification. As one of ordinary skill in the art will readily appreciate from the disclosure of the present disclosure, processes, machines, manufacture, compositions of matter, means, methods, or steps, presently existing or later to be developed, that perform substantially the same function or achieve substantially the same result as the corresponding embodiments described herein may be utilized according to the present disclosure. Accordingly, the appended claims are intended to include within their scope such processes, machines, manufacture, compositions of matter, means, methods, or steps.

**Claims**

1. A method for determining a safety index of a product, the method comprising:

   retrieving, by a computing system, a plurality of toxicity values of the product from a toxicity database, wherein the toxicity values correspond to a plurality types of the product respectively;
   retrieving, by the computing system, a plurality of weight values of the product from a weight database, wherein the weight values correspond to the types of the product respectively; and
   determining, by the computing system, the safety index of the product according to the toxicity values and the weight values.

2. The method of Claim 1, wherein a toxicity test is optionally performed to a plurality of samples of the types of the product to obtain the toxicity values, and the method further comprises:

   storing, by the computing system, the toxicity values in the toxicity database, optionally wherein

(a) the toxicity test includes an acute toxicity test and the toxicity values are associated with lethal concentration 50% ($LC_{50}$) values; or
(b) the toxicity test includes a chronic toxicity test and the toxicity values are associated with estradiol equivalent concentration (EEQ) values.

**3.** The method of Claim 1, wherein the weight values include sales volume information.

**4.** The method of Claim 1, wherein the safety index of the product is determined based on following formula:

$$\text{SI} = \sum_{h=1}^{x} \bar{T}_h \times \omega_h$$

wherein SI represents the safety index of the product, x represents number of the types of the product, $\bar{T}_h$ represents the toxicity value of $h^{th}$ type of the product, and $\omega_h$ represents the weight value of $h^{th}$ type of the product.

**5.** The method of Claim 4, wherein $\bar{T}_h$ is determined based on following formula:

$$\bar{T}_h = \frac{1}{N_h} \sum_{i=1}^{N_h} T_{hi}$$

wherein Nh represents number of samples of $h^{th}$ type of the product and Thi represents a toxicity test value of $i^{th}$ sample of $h^{th}$ type of the product.

**6.** The method of Claim 5, wherein $\bar{T}_h$ is transformed to $\bar{T}_H$ based on following formulas:

$$T_{hi}^* = \frac{T_{hi} - \min_{1 \le i \le N_h} T_{hi}}{\max_{1 \le i \le N_h} T_{hi} - \min_{1 \le i \le N_h} T_{hi}} \times 100\%$$

$$\bar{T}_H = \frac{1}{N_h} \sum_{i=1}^{N_h} T_{hi}^*$$

**7.** The method of Claim 4, wherein $\omega_h$ is determined based on following formula:

$$\omega_h = \frac{\sum_{i=1}^{N_h} C_{hi} P_{hi}}{\sum_{h=1}^{x} \sum_{i=1}^{N_h} C_{hi} P_{hi}}$$

wherein Nh represents number of samples of $h^{th}$ type of the product, $C_{hi}$ represents an amount of $i^{th}$ sample of $h^{th}$ type of the product and $P_{hi}$ represents a rank weight of $i^{th}$ sample of $h^{th}$ type of the product.

**8.** The method of Claim 7, wherein the rank weights are based on product popularities which reflect the sales volume information to a certain extent.

**9.** A computing system for determining a safety index of a product, the computing system comprising:

a processor; and

a memory storing instructions that, when being executed, cause the processor to:

retrieve a plurality of toxicity values of the product from a toxicity database, wherein the toxicity values correspond to a plurality types of the product respectively;

retrieve a plurality of weight values of the product from a weight database, wherein the weight values correspond to the types of the product respectively; and

determine the safety index of the product according to the toxicity values and the weight values.

10. The computing system of Claim 9, wherein a toxicity test is optionally performed to a plurality of samples of the types of the product to obtain the toxicity values, and the instructions, when being executed, further cause the processor to:

store the toxicity values in the toxicity database, optionally wherein (a) the toxicity test includes an acute toxicity test and the toxicity values are associated with lethal concentration 50% ($LC_{50}$) values; or (b) the toxicity test includes a chronic toxicity test and the toxicity values are associated with estradiol equivalent concentration (EEQ) values.

11. The computing system of Claim 9, wherein the weight values include sales volume information.

12. The computing system of Claim 9, wherein the safety index of the product is determined based on following formula:

$$\mathrm{SI} = \sum_{h=1}^{x} \bar{T}_h \times \omega_h$$

wherein SI represents the safety index of the product, x represents number of the types of the product, $\bar{T}_h$ represents the toxicity value of $h^{th}$ type of the product, and $\omega_h$ represents the weight value of $h^{th}$ type of the product.

13. The computing system of Claim 12, wherein $\bar{T}_h$ is determined based on following formula:

$$\bar{T}_h = \frac{1}{N_h} \sum_{i=1}^{N_h} T_{hi}$$

wherein $N_h$ represents number of samples of $h^{th}$ type of the product and $T_{hi}$ represents a toxicity test value of $i^{th}$ sample of $h^{th}$ type of the product.

14. The computing system of Claim 13, wherein $\bar{T}_h$ is transformed to $\bar{T}_H$ based on following formulas:

$$T_{hi}^* = \frac{T_{hi} - \min_{1 \leq i \leq N_h} T_{hi}}{\max_{1 \leq i \leq N_h} T_{hi} - \min_{1 \leq i \leq N_h} T_{hi}} \times 100\%$$

$$\bar{T}_H = \frac{1}{N_h} \sum_{i=1}^{N_h} T_{hi}^*$$

15. The computing system of Claim 12, wherein $\omega_h$ is determined based on following formula:

$$\omega_h = \frac{\sum_{i=1}^{N_h} C_{hi} P_{hi}}{\sum_{h=1}^{x} \sum_{i=1}^{N_h} C_{hi} P_{hi}}$$

wherein $N_h$ represents number of samples of $h^{th}$ type of the product, $C_{hi}$ represents an amount of $i^{th}$ sample of $h^{th}$ type of the product and $P_{hi}$ represents a rank weight of $i^{th}$ sample of $h^{th}$ type of the product, optionally wherein the rank weights are based on the product popularities which reflect the sales volume information to a certain extent.

FIG. 1A

FIG. 1B

FIG. 1C

Retrieving a plurality of toxicity values of a
product from a toxicity database

S201

Retrieving a plurality of weight values of the
product from a weight database

S202

Determining a safety index of the product
according to the toxicity values and the weight
values

S203

FIG. 2

```
┌─────────────────────────────────────────┐
│  Performing a toxicity test to a plurality of  │
│  samples of types of a product to obtain a  │
│  plurality of toxicity values        S301  │
└─────────────────────────────────────────┘
                    │
                    ▼
┌─────────────────────────────────────────┐
│   Storing the toxicity values in the toxicity  │
│                database              │
│                             S302  │
└─────────────────────────────────────────┘
                    │
                    ▼
┌─────────────────────────────────────────┐
│  Retrieving the toxicity values of the product  │
│         from the toxicity database       │
│                             S303  │
└─────────────────────────────────────────┘
                    │
                    ▼
┌─────────────────────────────────────────┐
│  Retrieving a plurality of weight values of the  │
│        product from a weight database      │
│                             S304  │
└─────────────────────────────────────────┘
                    │
                    ▼
┌─────────────────────────────────────────┐
│   Determining a safety index of the product   │
│  according to the toxicity values and the weight  │
│                 values              │
│                             S305  │
└─────────────────────────────────────────┘
```

# FIG. 3

Europäisches
Patentamt

European
Patent Office

Office européen
des brevets

# EUROPEAN SEARCH REPORT

Application Number

EP 19 18 5284

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/016430 A1 (GOUSTOVA GALINA [US]) 18 January 2007 (2007-01-18) * abstract; figure 1 * * paragraphs [0012], [0016] * ----- | 1-15 | INV. G06Q30/02 |

TECHNICAL FIELDS
SEARCHED      (IPC)

G06Q

The present search report has been drawn up for all claims

| Place of search | Date of completion of the search | Examiner |
|---|---|---|
| The Hague | 18 October 2019 | Fiorenzo Catalano, M |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 
& : member of the same patent family, corresponding
    document

EPO FORM 1503 03.82 (P04C01)

## EP 3 594 887 A1

**ANNEX TO THE EUROPEAN SEARCH REPORT
ON EUROPEAN PATENT APPLICATION NO.**

EP 19 18 5284

This annex lists the patent family members relating to the patent documents cited in the above-mentioned European search report.
The members are as contained in the European Patent Office EDP file on
The European Patent Office is in no way liable for these particulars which are merely given for the purpose of information.

18-10-2019

| Patent document cited in search report | Publication date | Patent family member(s) | Publication date |
|---|---|---|---|
| US 2007016430 A1 | 18-01-2007 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20170356895 A

- US 20170356900 A

**Non-patent literature cited in the description**

- *Analytica Chimica Acta,* 2016, vol. 915, 1-26